# EUROPEAN PATENT APPLICATION

(11) **EP 4 813 335 A1**
(43) Date of publication of application: **30.09.2026**
(21) Application number: 25165577.5
(22) Date of filing: 24.03.2025
(51) Int. Cl.: A61B 34/20, A61B 34/00, A61B 90/00, A61B 90/20

(54) **SYSTEM, METHOD, AND COMPUTER PROGRAM FOR A SURGICAL IMAGING SYSTEM, SURGICAL IMAGING SYSTEM**

(71) Applicant: Leica Instruments (Singapore) Pte Ltd., Singapore 618299 (SG)
(72) Inventor: THEMELIS, George, 618299 Singapore (SG)
(74) Representative: 2SPL Patentanwälte PartG mbB

(57) **Abstract**

Examples relate to a system, a method, and a computer program for a surgical imaging system, and to a surgical imaging system comprising such a system. The system is configured to obtain image-guided surgery data of an image-guided surgery system, the image-guided surgery data comprising three-dimensional anatomical information of a patient, obtain image data of at least one optical imaging sensor of the surgical imaging system, the image data showing a surgical site (10) of the patient and at least one surgical tool (20), determine a position of a portion of the surgical tool relative to the surgical site based on the image data, determine a distance between the position of the portion of the surgical tool and at least one anatomical feature (30) of the patient based on the image-guided surgery data, and provide a notification (40a, 40b) based on the distance between the position of the portion of the surgical tool and at least one anatomical feature of the patient.

## Description

### Technical field

Examples relate to a system, a method, and a computer program for a surgical imaging system, and to a surgical imaging system comprising such a system.

### Background

Surgeons frequently operate near critical anatomical structures such as vessels, nerves, and tumors. Accidental damage to these structures can lead to severe complications. Current intraoperative imaging systems can be used to display sub-surface structures. In particular, to enhance surgical precision, visual overlays of sub-surface structures are displayed. These structures are displayed on the surgical view using advanced visualization techniques, which often rely on an Image-Guidance System (IGS) for accurate spatial alignment. Surgeons must continuously monitor the visual displays, thereby increasing their cognitive load. Moreover, there are no active alerts or warnings when tools come close to critical structures, and the presence of visual clutter can obscure important details.

Auditory alerts based on predefined zones are a known concept in surgical settings. Some systems provide audio notifications when instruments enter certain predefined zones. The predefined zones are static and may not accurately reflect the real-time position of tools relative to dynamic anatomical structures. Additionally, these alerts lack specificity, as the surgeon is unable to distinguish which critical structure is being approached.

In the field of robotic surgery, haptic feedback is utilized, with robotic systems providing force feedback when instruments make contact with specific tissues. This approach may not be applicable to manual surgical instruments and may necessitate the use of costly robotic systems.

There may be a desire for an improved mechanism for avoiding damage to critical anatomical structures during surgery.

### Summary

This desire is addressed by the subject matter of the independent claims.

Various examples of the present disclosure are based on the finding that the position of critical anatomical structures, such as vessels, nerves, and tumors, is often known from Image-Guided Surgery (IGS) systems. Image-guided surgery is a surgical tool that utilizes pre-operative and intra-operative imaging to guide the surgical instruments and plan the operation more accurately. In image-guided surgery data, anatomical structures are represented in a detailed three-dimensional model of the patient's anatomy (which can be based on Magnetic Resonance Imaging, Computed Tomography, or ultrasound imaging). This three-dimensional model can be used to aid in navigating the operative field precisely. In the context of the present disclosure, the IGS data is used for the purpose of determining a distance between a surgical tool and at least one anatomical feature of the patient, with image processing being used to determine the position of a portion (such as the tip or blade) of the surgical tool. Based on the distance, a notification is provided, e.g., if the surgical tool approaches the at least one anatomical feature. This way, the surgeon can be warned to be particularly careful near (critical) anatomical features.

Some aspects of the present disclosure relate to a system for a surgical imaging system. The system comprises one or more processors and one or more storage devices. The system is configured to obtain image-guided surgery data of (e.g., from) an image-guided surgery system. The image-guided surgery data comprises three-dimensional anatomical information of (e.g., about) a patient. The system is configured to obtain image data of at least one optical imaging sensor of the surgical imaging system. The image data shows a surgical site of or on the patient and at least one surgical tool. The system is configured to determine a position of a portion of the surgical tool relative to the surgical site based on the image data. The system is configured to determine a distance between the position of the portion of the surgical tool and at least one anatomical feature of the patient based on the image-guided surgery data. The system is configured to provide a notification based on the distance between the position of the portion of the surgical tool and at least one anatomical feature of the patient. This way, the surgeon can be warned to be particularly careful near (critical) anatomical features.

In general, IGS data is three-dimensional data, i.e., it represents the extent of the anatomical features, at and below the surface of the surgical site. Thus, the system may be configured to determine the distance between the position of the portion of the surgical tool and at least one anatomical feature of the patient located below a surface of the surgical site based on the image-guided surgery data. This way, notifications can be provided for anatomical features that cannot be seen by the surgeon (as they are below the surface), so that the surgeon can be warned when he or she approaches the anatomical feature.

The same holds true for the surgical tool - the proposed concept can be used to provide notifications regardless of whether the portion of the surgical tool is above the surface or immersed in the tissue. In other words, the system may be configured to determine the position of the portion of the surgical tool regardless of whether the portion of the surgical tool is above or below the surface of the surgical site. In this case, the position of the portion of the surgical tool can be extrapolated from the position and orientation of the remainder of the surgical tool that is above the surface.

For example, the portion of the surgical tool may be one of a tip of the surgical tool, a blade of the surgical tool (e.g., of a scalpel), or a tube opening (e.g., an opening of a surgical aspirator or suction device). In more general terms, the portion of the surgical tool may be the portion of the surgical tool that is closest to the surgical site, i.e., as the surgeon uses this portion to perform the surgical procedure.

In some cases, non-anatomical objects, such as clips, may be placed on the surgical site to close blood vessels during the procedure. They help control bleeding by clamping and sealing off the vessels, ensuring that the surgical area remains dry and blood-free. Clips can also be used for securing tissues or organs in place temporarily during surgery. To avoid interfering with non-anatomical clips, the notification system may be extended to non-anatomical objects as well. In other words, the system may be configured to determine a position of a non-anatomical object relative to the surgical site based on the image data. The system may be configured to determine a distance between the position of the portion of the surgical tool and the position of the non-anatomical object. The system may be configured to provide the notification based on the distance between the position of the portion of the surgical tool and the position of the non-anatomical object. This way, contact between the surgical tool and non-anatomical objects may be avoided.

In many cases, it is useful for the surgeon to distinguish between different types of non-anatomical objects based on the notification being provided. Accordingly, the system may be configured to provide a notification having a first notification configuration based on the distance between the position of the portion of the surgical tool and the position of a first non-anatomical object and a second notification having a second notification configuration based on the distance between the position of the portion of the surgical tool and the position of a second non-anatomical object. This way, the surgeon can distinguish between different instances or types of non-anatomical objects from the notification alone.

The same approach may be taken for anatomical features, where it is also useful for the surgeon to distinguish between different anatomical features, e.g., different types of anatomical features, based on the notification being provided. Accordingly, the system may be configured to provide a notification having a first notification configuration based on the distance between the position of the portion of the surgical tool and a first anatomical feature, and a second notification having a second notification configuration based on the distance between the position of the portion of the surgical tool and a second anatomical feature. This way, the surgeon can distinguish between different anatomical features from the notification alone.

To avoid having to manually define which notification configuration is used for which anatomical feature or non-anatomical object, the notification configurations may be selected according to a categorization of the respective anatomical feature or non-anatomical object. For example, the system may select one notification configuration for blood vessels and another notification configuration for tumors, one notification configuration for surgical clips and another for sponges. For example, the system may be configured to select one of the first notification configuration or the second notification configuration based on a category of the respective surgical tool, anatomical feature, or non-anatomical object, with the category being one of a plurality of pre-defined categories. For example, with respect to anatomical features, the plurality of pre-defined categories may comprise at least a first category used for tissue to be removed (e.g., for tumors) and a second category used for blood vessels. With respect to non-anatomical objects, the plurality of pre-defined categories may comprise at least a first category used for features that are to remain static (such as clips, clamps etc.) and a second category used for features that can be moved (such as surgical sponges). With respect to surgical tools, the plurality of pre-defined categories may comprise at least a first category used for surgical cutting tools (such as scalpels, drills or saws) and a second category used non-cutting tools (such as forceps, suction devices).

As an alternative to, or in addition to, using a categorization-based approach, the notification configuration may be assigned manually to anatomical features and/or non-anatomical objects. In other words, the system may be configured to obtain an input from a user of the surgical imaging system. The input may comprise a mapping between at least one anatomical feature or non-anatomical object and at least one notification configuration. The system may be configured to select one of the first notification configuration or the second notification configuration based on the mapping. This way, anatomical features or non-anatomical objects that are of particular interest can be highlighted with a custom notification configuration, further raising awareness of the distance between the respective object and surgical tool.

There are various aspects of a notification that can be changed to enable the surgeon to distinguish between different notifications. For example, a notification configuration may specify at least one of a color to be used for a visual overlay, a color to be used for a notification icon, the distance being included in a visual overlay (i.e., whether the distance is to be included in the visual overlay, and/or a unit to be used, such as mm), an alarm tone to be used for an audio output, and a haptic pattern to be used for a haptic notification. Accordingly, notification configurations may differ with respect to at least one of the color to be used for the visual overlay, the color to be used for the notification icon, the distance being included in the visual overlay, the alarm tone to be used for the audio output, and the haptic pattern to be used for the haptic notification. This way, the surgeon can distinguish different anatomical features and/or non-anatomical objects based on the notification configuration of the notification provided.

During surgery, visual clutter or undesired audio or haptic notifications may distract the surgeon. If the surgeon is still at a certain distance from the object (e.g., anatomical feature or non-anatomical object), he or she might not want to be (overly) bothered with a notification. Therefore, the noticeability of the notifications may be varied (e.g., scaled) based on the distance, so that the notification becomes more noticeable as the distance decreases. Thus, the system may be configured to vary (e.g., scale) at least one of an intensity (e.g., a sound intensity of an audio notification, a haptic intensity of a haptic notification, or a color intensity of a visual notification), a size (e.g., of a visual notification) or a transparency (e.g., of a visual notification) of the notification based on the distance. This way, the cognitive load on the surgeon may be reduced for cases in which the distance is non-critical.

Similarly, notifications might only be provided if the surgical tool comes close enough to the object in question. In other words, the system may be configured to provide the notification based on a comparison of the distance with a distance threshold. This way, visual clutter or undesired audio or haptic notifications may be avoided for cases in which the distance is non-critical.

For example, the system may be configured to use different distance thresholds for different categories of surgical tools, anatomical features or non-anatomical objects. As different categories of objects are of different criticality, this is another way to avoid unnecessary notifications for non-critical objects.

In various examples, visual notifications may be used. For example, the system may be configured to generate a view on the surgical site based on the image data, with the view including an overlay with the notification. The system may be configured to provide the view on the surgical site for a display device of the surgical imaging system. This way, the surgeon can visually perceive the notifications while performing surgery using the same view, enabling the surgeon to directly perceive the object having caused the notification.

Again, to avoid visual clutter, visual notifications may be shown only if the surgical tool comes close enough to the respective object. In other words, the system may be configured to include the overlay in the view on the surgical site based on a comparison of the distance with a distance threshold.

To help the surgeon better understand which surgical tool, anatomical feature or non-anatomical feature has caused the notification, the notification may be overlaid over the respective surgical tool, anatomical feature or non-anatomical object, mimicking the shape of the respective surgical tool, anatomical feature, or non-anatomical object. In other words, a shape of the overlay may be based on at least one of a shape or projected shape of the surgical tool or a projected shape of the anatomical feature or non-anatomical object. This way, the surgeon can better identify the cause of the notification.

In addition to, or as an alternative to, visual notifications, audio notifications may be used. In other words, the system may be configured to provide an audio output (e.g., a spoken notification or a beeping notification similar to parking sensors in vehicles) comprising the notification. This way, the field of view of the surgeon can remain less cluttered.

Another notification option is haptic notifications. In other words, the system may be configured to provide a control signal for a haptic notification device, with the control signal comprising the notification. This is another way in which the field of view of the surgeon can remain less cluttered.

In the proposed concept, the position of the anatomical features is provided by the IGS system. The position of the portion of the surgical tool (and, optionally, of the non-anatomical objects), are determined based on the image data. Various techniques may be used for this purpose. For example, the system may be configured to process the image data using a machine-learning model to estimate a pose of the surgical tool and to determine the position of the portion of the surgical tool based on the estimated pose of the surgical tool. For example, a machine-learning model may process the image data to estimate the pose (position and orientation) of the entire surgical tool. Once the overall pose is estimated, the system can then calculate the precise location of the desired portion of the tool based on its known geometry and dimensions relative to the estimated pose. This allows for accurate tracking of critical parts of the surgical tool in real-time.

Another technique that can be used is based on fitting a projection of a 3D model of the surgical tool onto the surgical tool shown in the image data. In other words, the system may be configured to determine the position of the portion of the surgical tool by fitting a projection of a three-dimensional model of the surgical tool to the surgical tool shown in the image data. To achieve this, the system may be configured to generate 2D projections of the three-dimensional model from various angles, and compare these projections with the image data to find the best match. The angle and position that yield the closest fit indicate the orientation and location of the surgical tool's portion in question (e.g., the tip or blade). This process allows for accurate determination of the tool's position, even when only a partial view is available.

Another aspect of the present disclosure relates to a surgical imaging system comprising the above system. In some examples, the surgical imaging system may further comprise the IGS system.

Some aspects of the present disclosure relate to a corresponding method for a surgical imaging system. The method comprises obtaining image-guided surgery data of an image-guided surgery system. The image-guided surgery data comprises three-dimensional anatomical information of a patient. The method comprises obtaining image data of at least one optical imaging sensor of the surgical imaging system. The image data shows a surgical site of the patient and at least one surgical tool. The method comprises determining a position of a portion of the surgical tool relative to the surgical site based on the image data. The method comprises determining a distance between the position of the portion of the surgical tool and at least one anatomical feature of the patient based on the image-guided surgery data. The method comprises providing a notification based on the distance between the position of the portion of the surgical tool and at least one anatomical feature of the patient. Another aspect of the present disclosure relates to a computer program having a program code for performing the above method when the program is executed on processor.

### Short description of the Figures

Some examples of apparatuses and/or methods will be described in the following by way of example only, and with reference to the accompanying figures, in which:
- Fig. 1a: shows a block diagram of an example of a system for a surgical imaging system;
- Fig. 1b: shows a schematic diagram of an example of a surgical imaging system;
- Fig. 1c: shows a schematic drawing of a visualization of a distance between a surgical tool and a tumor;
- Fig. 2: shows an image of an example of real-time proximity warning for cancerous tissue;
- Fig. 3: shows a flow chart of an example of a method for a surgical imaging system; and
- Fig. 4: shows a schematic diagram of an example of a system comprising an imaging device and a computer system.

### Detailed Description

Various examples will now be described more fully with reference to the accompanying drawings in which some examples are illustrated. In the figures, the thicknesses of lines, layers and/or regions may be exaggerated for clarity.

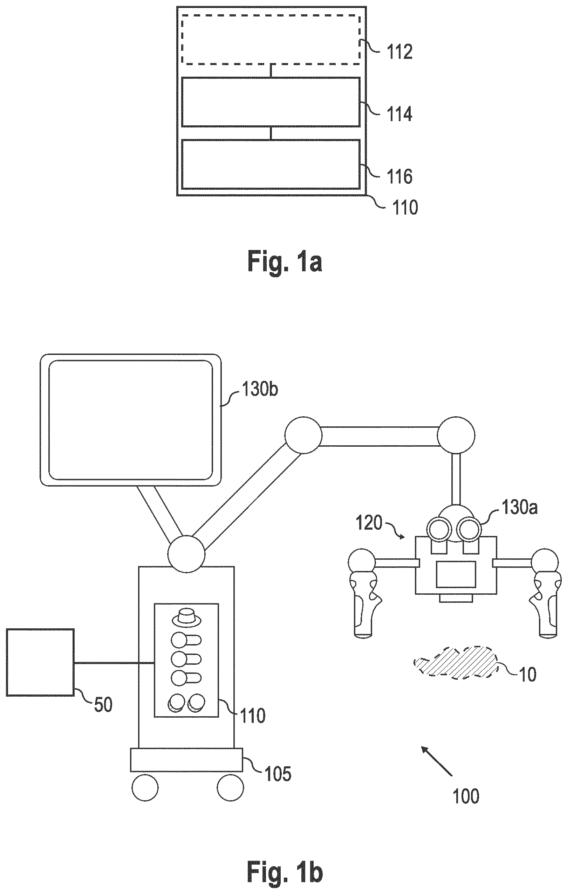

Fig. 1a shows a schematic diagram of an example of a system 110 for a surgical imaging system 100 (shown in Fig. 1b). The system 110 is a component of the surgical imaging system 100 and may be used to control various aspects of the surgical imaging system 100. In particular, it may be used for acquisition and/or processing of imaging data by at least one optical imaging sensor of an imaging device 120 of the surgical imaging system, through various means that will be introduced in more detail in the following. In addition, the system 110 may be configured to control additional aspects of the surgical imaging system 100, e.g., to provide a display signal for one or more display devices 130a, 130b of the surgical imaging system, and/or to control and/or execute automatic operations of the surgical imaging system, e.g., using an automated imaging device positioning system, such as a robotic arm.

In general, the system 110 may be a computer system. The system 110 comprises one or more processors 114 and one or more storage devices 116. Optionally, the system 110 further comprises one or more interfaces 112. The one or more processors 114 are coupled to the one or more storage devices 116 and to the one or more interfaces 112. In general, the functionality of the system 110 may be provided by the one or more processors 114, in conjunction with the one or more interfaces 112 (for exchanging data/information with one or more other components of the surgical imaging system 100 and outside the surgical imaging system 100, such as an optical imaging sensor of the imaging device 120 or an automated imaging device positioning system 120), and with the one or more storage devices 116 (for storing information, such as machine-readable instructions of a computer program being executed by the one or more processors). In general, the functionality of the one or more processors 114 may be implemented by the one or more processors 114 executing machine-readable instructions. Accordingly, any feature ascribed to the one or more processors 114 may be defined by one or more instructions of a plurality of machine-readable instructions. The system 110 may comprise the machine-readable instructions, e.g., within the one or more storage devices 116.

As outlined above, the system 110 is part of the surgical imaging system 100, which comprises various components in addition to the system 110. For example, the surgical imaging system 100 comprises the imaging device 120, and may comprise one or more additional components, such as one or more display devices 130a, 130b, such as ocular displays 130a and/or an auxiliary display 130b. Fig. 1b shows a schematic diagram of an example of such a surgical imaging system 100, and in particular of a surgical microscope system 100. In the following, the surgical imaging system 100 may also be referred to as surgical microscope system 100. A surgical microscope system is a surgical imaging system 100 that comprises a (surgical) microscope as imaging device 120. However, the proposed concept is not limited to such embodiments. The surgical imaging system 100 may be based on various (single or multiple) imaging devices, such as one or more microscopes, one or more endoscopes, and/or one or more exoscopes (also sometimes called an extracorporeal telescope). Exoscopes are camera-based imaging systems, and in particular camera-based 3D imaging systems, which are suitable for providing images of surgical sites with high magnification and a large depth of field. Compared to microscopes, which may be used via oculars 130a, exoscopes are only used via display modalities, such as a monitor or a head-mounted display. Accordingly, the surgical imaging system 100 may alternatively be a surgical endoscope system, or a surgical exoscope system. The following illustrations assume that the surgical imaging device 120 is a surgical microscope, and that the surgical imaging system 100 is a surgical microscope system 100.

Accordingly, the surgical imaging system or surgical microscope system 100 may comprise an imaging device, such as a microscope 120. In general, a microscope, such as the microscope 120, is an optical instrument that is suitable for examining objects that are too small to be examined by the human eye (alone). For example, a microscope may provide optical magnification of a sample, such as a surgical site. In the present concept, the optical magnification is (also) provided for at least one optical imaging sensor. The microscope 120 thus comprises an optical imaging sensor, which is coupled with the system 110. The microscope 120 may further comprise one or more optical magnification components that are used to magnify a view on the sample, such as an objective (i.e., lens). For example, the surgical imaging device or microscope 120 is often referred to as the 'optics carrier' of the imaging system.

There are a variety of different types of surgical imaging devices. If the imaging device is used in the medical or biological fields, the object being viewed through the imaging device may be a sample of organic tissue, e.g., arranged within a petri dish or present in a part of a body of a patient. In various examples presented here, the imaging device 120 may be a microscope of a surgical microscope system, i.e., a microscope that is to be used during a surgical procedure, such as a neurosurgical procedure (i.e., brain surgery). Accordingly, the sample being viewed through the surgical imaging device may be a sample of organic tissue of a patient and may in particular be the surgical site that the surgeon operates on during the surgical procedure, e.g., the brain. However, the proposed concept is also suitable for other types of surgery, such as eye surgery or cardiac surgery.

Fig. 1b shows a schematic diagram of an example of a surgical imaging system 100, and in particular of a surgical microscope system 100, comprising the system 110 and a microscope 120. The surgical microscope system 100 shown in Fig. 1b comprises a number of optional components, such as a base unit 105 (comprising the system 110) with a (rolling) stand, ocular displays 130a that are arranged at the imaging device 120, an auxiliary display 130b that may be arranged at the base unit, and the arm 120 that holds the microscope 120 in place, and is coupled to the base unit and to the microscope 120. In Fig. 1b, the system 110 is coupled with an IGS system 50.

The proposed concept relates to warning the surgeon for the proximity of surgical tools to critical structures (depth warning). In the proposed concept, the system 110 is used for the purpose of providing a notification based on the distance, at a surgical site, between a surgical tool and anatomical feature(s) of the surgical site. While other intraoperative imaging systems may display sub-surface structures, they do not actively alert the surgeon when surgical tools are in close proximity to these critical areas. The proposed concept provides a system that, utilizing an Image-Guided Surgery (IGS) system, measures the distance between surgical tools and critical sub-surface structures in real-time and provides immediate, intuitive feedback to the surgeon to prevent inadvertent injury. For this purpose, the system 110 uses image-guided surgery data of an image-guided surgery system 50 and image data showing the surgical site. From the image data of the surgical site, the position of a portion of the surgical tool (e.g., its tip, blade, or opening of an aspirator or suction device) is determined and used to determine the distance to the anatomical feature(s) at the surgical site. Based on the distance, a notification is provided.

The process starts with the IGS data. The system is configured to obtain the image-guided surgery data of (e.g., from) the image-guided surgery system 50. Image Guided Surgery (IGS) is a modern surgical technique that uses real-time imaging to help surgeons navigate precisely during procedures. In the present context, the IGS system is used for tracking the spatial positions of various elements, including the patient, microscope, sub-surface structures, and surgical tools. By doing so, the IGS system provides the necessary spatial information to calculate distances between surgical tools and critical structures with high accuracy. IGS data consists of medical imaging information, typically from CT scans, MRI, or other imaging modalities, which is processed and displayed to provide surgeons with detailed anatomical mapping during surgery. This data creates a virtual, three-dimensional representation of the surgical site, allowing surgeons to visualize their instruments' position in relation to the patient's anatomy in real-time. In other words, the image-guided surgery data comprises three-dimensional anatomical information of the patient, and in particular of the anatomical site. In the proposed concept, this three-dimensional representation is used to determine the position and extent of anatomical features of interest, such as blood vessels, tumors, or nerves for comparison with the position of certain portions of the surgical tools. Critical structure mapping is achieved by integrating the preoperative 3D imaging data, such as MRI or CT scans, included in the IGS data. This helps in identifying the locations of critical sub-surface structures. The IGS system may align this data with the intraoperative 3D model to ensure accurate spatial correlation.

In the proposed concept, the system 110 is configured to determine a distance between a position of a portion of the surgical tool (such as its tip, blade, or opening) and at least one anatomical feature 30 of the patient based on the image-guided surgery data. In some examples, the distance measurement may be based on 3D scanning of the surgical field. This scan can be performed using multiple methods. One implementation may utilize the microscope's stereo imaging capabilities. By analyzing the disparity in these stereo images, a real-time 3D model of the surgical field may be generated, including the position of the portion of the surgical tool relative to the surgical site (and thus the anatomical feature(s), as represented in the IGS data). Additionally, or alternatively, other 3D scanning techniques such as structured light scanning or time-of-flight sensors can also be employed depending on the system's capabilities.

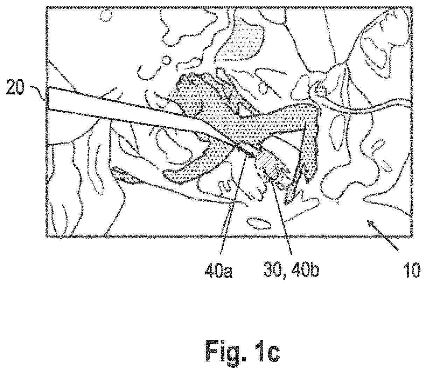

The position of the surgical tool is determined using image data of the surgical imaging device 120. Thus, the system 110 is configured to obtain image data of at least one optical imaging sensor of the surgical imaging system, with the image data showing the surgical site 10 of the patient and at least one surgical tool 20 (see Fig. 1c). Fig. 1c shows a schematic drawing of a visualization 40a of a distance between the surgical tool 20 and a tumor 30. In addition, a visualization 40b is provided of the tumor itself, as the tumor is below the surface of the surgical site 10.

As can be seen in Fig. 1c, the surgical tool can be clearly seen in the image data. This means that the position of the tip, blade or tube opening can be determined visually. The system 110 is configured to determine a position (and, optionally, an orientation) of a portion of the surgical tool relative to the surgical site based on the image data. This can be done using various mechanisms.

A first mechanism is based on the use of machine learning. In other words, tool detection and localization are facilitated through Al (Artificial Intelligence)-based methods. Artificial Intelligence algorithms may be used to detect and recognize surgical tools within the imaging field shown in the image data. These algorithms may be used to determine the position and orientation of the portion of the tool, such as its tip, even when it is partially obscured or immersed in tissue. For example, given that a limited number of surgical tools (scalpel, forceps, scissors, retractor, hemostat, surgical drill, surgical saw, suction device, etc.) are being used during surgery, a machine learning model may be trained to predict the position of the portion of the surgical tool. In particular, pose estimation may be used for this purpose. In other words, the system may be configured to process the image data using a machine-learning model to estimate a pose of the surgical tool, and to determine the position of the portion of the surgical tool based on the estimated pose of the surgical tool.

Pose estimation is a computer vision technique that determines the position and orientation of objects in 2D or 3D space from image data. In surgical applications, it involves detecting and tracking the spatial coordinates, rotation angles, and overall configuration of surgical instruments, anatomical structures, or other relevant objects within the field of view. For surgical imaging applications, pose estimation using machine learning typically involves deep neural networks. These models process the microscope's image data by identifying key features and landmarks of the target object. The network can be designed to output either direct pose parameters (position and orientation) or intermediate representations like keypoints that can be used to derive the pose.

Training such a machine learning model requires a large dataset of labeled images showing the objects of interest from various angles, positions, and lighting conditions. The training data further includes ground truth pose information for each image. This can be obtained through manual annotation, synthetic data generation, or using other sensing systems as reference. The model learns to associate visual features with corresponding pose parameters through supervised learning, minimizing the difference between predicted and actual poses.

To determine the position of a specific portion of a surgical tool, such as a scalpel blade, the system can combine the pose estimation results with known object dimensions and imaging device parameters, such as zoom level and working distance. Using the overall pose as a starting point, the position of any point on the object can be calculated using geometric transformations. The microscope's zoom factor and working distance are used for converting image coordinates of the image data to real-world coordinates. By applying scaling factors according to the zoom factor and working distance and using the known dimensions of the surgical instrument, the system can compute the location of specific features like the scalpel blade tip or edges as position of the portion of the surgical tool.

In general, for a machine learning model trained on a known, limited number of different surgical tools, this approach also works if a portion of the surgical tool is not visible (e.g., obstructed or outside the field of view) in the image data, as the machine learning model can be trained to estimate the pose of the surgical tool even based on image data where some portions of the surgical tool cannot be seen. This enables determination of the position of the portion of the surgical tool even if that portion of the surgical tool cannot be seen (e.g., as it is below the surface of the surgical site). Thus, the system may be configured to determine the position of the portion of the surgical tool regardless of whether the portion of the surgical tool is above or below the surface of the surgical site. In other words, Al algorithms may be used for obscured tool tip estimation to estimate the position of the portion of the surgical tool even when it is immersed in tissue or obstructed, ensuring accurate distance measurements.

A second technique for determining the position of the portion of the surgical tool uses a 3D model of the surgical tool to determine the pose, i.e., position and orientation, of the surgical tool. In particular, the system may be configured to determine the position of the portion of the surgical tool by fitting a projection of a three-dimensional model of the surgical tool to the surgical tool shown in the image data. The projection of a three-dimensional model of a surgical tool onto the image data can be accomplished through a process called model-based tracking. By creating a virtual projection of the three-dimensional model of the tool and overlaying it onto the 2D microscope image, an optimization algorithm can be used to adjust the position and orientation of the projected model until it best matches the actual tool visible in the image. This fitting process typically involves reducing or minimizing the difference between the projected model's edges and the corresponding edges detected in the image data. The algorithm iteratively refines the tool's pose parameters (position and orientation) until the projected model closely aligns with the real tool's image.

Once the projection is accurately fitted to the surgical tool in the image data, the position of the portion of the tool (like a scalpel blade) can be determined using the known zoom factor and working distance of the microscope. Since these optical parameters define the relationship between real-world dimensions and image coordinates, they allow for the conversion of the fitted model's coordinates in the image space back to actual physical coordinates in three-dimensional space. The working distance provides the depth information, while the zoom factor helps establish the correct scale for translating pixel measurements to real-world dimensions. This transformation yields the position of the portion of the surgical tool in physical space.

The resulting position of the portion of the surgical tool and the position and extent of the anatomical feature(s) are then used to determine the distance between the position of the portion of the surgical tool 20 and the at least one anatomical feature 30 of the patient. For example, the distance may be determined in a three-dimensional world coordinate system, e.g., a coordinate system used by the IGS system. Distance calculation may be performed in real-time. In particular, computing the distance between the portion of the tool and nearby critical structures (i.e., anatomical structures) is performed using spatial data from the IGS system. As the position and extent of the anatomical feature(s) below the surface are known from the IGS data, the system may determine the distance between the position of the portion of the surgical tool and at least one anatomical feature of the patient being located below a surface of the surgical site based on the image-guided surgery data, regardless of whether the portion of the surgical tool is above or below the surface of the surgical site.

In some examples, not only is the distance between the surgical tool and anatomical features of interest, but also the distance between the surgical tool and other non-anatomical objects, such as surgical clips. Therefore, using techniques similar those being used to determine the position of the portion of the surgical tool, the position of such non-anatomical objects may be determined as well. In other words, the system may be configured to determine a position (e.g., and an extent) of a non-anatomical object relative to the surgical site based on the image data, and to determine a distance between the position of the portion of the surgical tool and the position of the non-anatomical object.

Various feedback mechanisms may be used to enhance the surgical process. In particular, the system 110 is configured to provide a notification 40a, 40b based on the distance between the position of the portion of the surgical tool and at least one anatomical feature of the patient, and/or based on the distance between the position of the portion of the surgical tool and the position of the non-anatomical object. In this context, a notification is a piece of information that is provided to the surgeon to inform the surgeon of the determined distance. In the following, three major categories of notifications are discussed.

A first category of notifications is visual notifications. In other words, the notification may provide visual feedback on the distance. For this purpose, the visual notification may be overlaid over a view of the surgical site (as used by the surgeon during surgery). For example, the system 110 may be configured to generate a view on the surgical site based on the image data, with the view including an overlay with the notification. The system may be configured to provide the view on the surgical site for a display device 130a, 130b of the surgical imaging system. In the following, some examples of such an overlay are shown.

Fig. 1c shows a schematic drawing visualizing the distance between a surgical tool and a tumor. In Fig. 1c, the overlay includes an arrow 40a that is used to indicate the distance between the surgical tool 20 and the tumor 30. The overlay further includes a visualization of a projected shape 40b of the anatomical feature (i.e., a 3D-to-2D projection of the anatomical feature's shape).

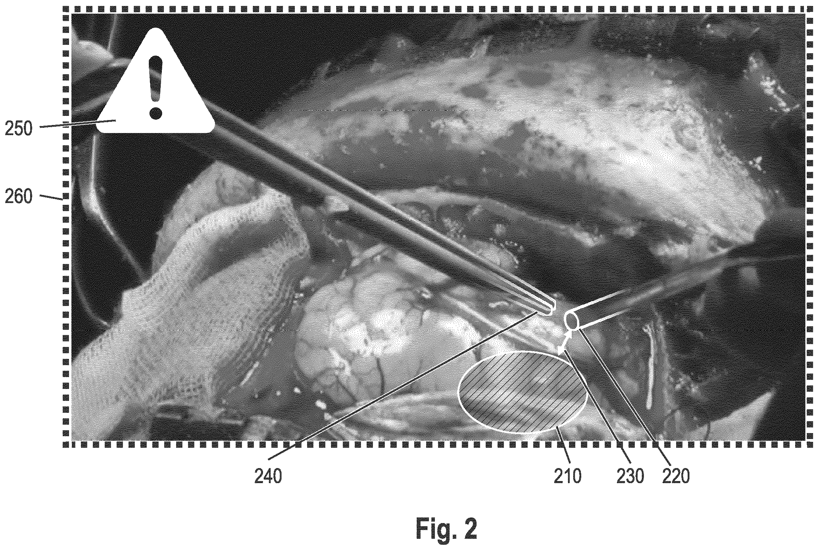

Fig. 2 shows an image of an example of real-time proximity warning for cancerous tissue. Fig. 2 illustrates a system that provides real-time feedback as surgical tools 220, 240 approach critical structures 210, specifically cancerous tissue. Visual alerts include a warning sign 250 in the upper left and a highlighted image border 260 to signal proximity to the cancer. The system integrates with the IGS platform to enhance safety during surgery by actively warning the surgeon of potential risks. In Fig. 2, in the background, the view on the surgical site is shown. In this case, the overlay includes the projected shape 210 of a tumor as well as highlighted edges 220, 240 of a suction device (220) and forceps (240). The overlay further includes an arrow 230 representing the distance between the suction device 220 and the tumor 210. In the upper left warning, the overlay includes a display icon 250 to alert the surgeon of the proximity between the suction device 220 and the tumor 210. The overlay further includes a (flashing) color frame 260 around the view of the surgical site.

As can be seen in Fig. 2, on-screen indicators, such as display icons 250 or flashing color frames 260 around the surgical image may be included in the overlay when approaching critical structures. Additionally, Augmented Reality (AR) overlays 220, 240 may change the color of the instrument or its tip in the display (e.g., in the overlay). The AR overlay is based on the shape of the surgical tool(s), anatomical feature(s) and/or non-anatomical object(s). Thus, a shape of (a portion of) the overlay may be based on at least one of a shape or projected shape of the surgical tool or a projected shape of the anatomical feature or non-anatomical object. The color, intensity, or flashing frequency may depend on the distance to the critical structure. In other words, the system may be configured to vary at least one of an intensity, a size, or a transparency of the notification based on the distance. This is also applicable to audio feedback and haptic feedback.

In some examples, adaptive visualization techniques can be employed. For example, critical structures may be faintly shown or hidden until the tool approaches them. More generally, the system may be configured to include the overlay (e.g., an overlay of the projected shape of an anatomical feature) in the view on the surgical site based on a comparison of the distance with a distance threshold. Upon approaching these (anatomical) structures, they can be revealed or highlighted, and may change color to indicate proximity.

Another type of notification is audio notifications. For example, audio feedback may be provided through auditory alerts. Accordingly, the system may be configured to provide an audio output comprising the notification, such as a spoken notification or a notification beep.

These alerts/notifications may vary in intensity, frequency, or repetition rate depending on the proximity (i.e., distance) to a critical structure.

A third type of feedback is haptic feedback. For example, the system may be configured to provide a control signal for a haptic notification device, with the control signal comprising the notification. For example, a wearable device may be used as a haptic notification device. Tactile sensations may be provided through devices such as bracelets or head-mounted displays. Alternatively, or additionally, contactless methods may be used. For example, the haptic notification device may comprise one or more focusable, directional ultrasound devices to deliver haptic sensations without direct contact. Alternatively, or additionally, instrument-based feedback may be used. For example, the respective surgical tools may comprise vibrating components as haptic notification device to provide feedback directly through the instrument.

In general, the surgeon benefits from being able to differentiate between different structures based on the notifications provided. For example, feedback types or patterns can be assigned to different critical structures such as vessels, nerves, and tumors. More generally, for different anatomical features and/or non-anatomical objects, different notifications (i.e., notifications having different notification configurations) may be used. In other words, the system may be configured to provide a notification having a first notification configuration based on the distance between the position of the portion of the surgical tool and the position of a first non-anatomical object or anatomical feature, and a second notification having a second notification configuration based on the distance between the position of the portion of the surgical tool and the position of a second non-anatomical object or anatomical feature. For example, notification configurations may differ with respect to at least one of a color to be used for a visual overlay, a color to be used for a notification icon, the distance being included in a visual overlay, an alarm tone to be used for an audio output, or a haptic pattern to be used for a haptic notification.

In many cases, presets can be used to select an appropriate notification configuration based on a categorization of the respective surgical tool, anatomical feature or non-anatomical object. For example, object recognition, such as tool recognition may be used, in which Al detects and identifies different surgical instruments, adjusting feedback accordingly. In other words, the system may be configured to use an object detection machine learning model to detect and determine a category of the surgical tool and/or the non-anatomical object. Moreover, the system may be configured to use the IGS data to determine the category of the anatomical feature. The system may then select one of the first notification configuration or the second notification configuration based on the category of the respective surgical tool, anatomical feature, or non-anatomical object, the category being one of a plurality of pre-defined categories. As outlined above, with respect to anatomical features, the plurality of pre-defined categories may comprise a category used for tissue to be removed (e.g., for tumors) and a category used for blood vessels. With respect to non-anatomical objects, the plurality of pre-defined categories may comprise a category used for features that are to remain static (such as clips, clamps etc.) and a category used for features that can be moved (such as surgical sponges). With respect to surgical tools, the plurality of pre-defined categories may comprise a category used for surgical cutting tools (such as scalpels, drills or saws) and a category used for non-cutting tools (such as forceps, suction devices).

In addition (or as an alternative) to the category-based selection of the notification configurations, the surgeon (or an assistant) may manually define a mapping between anatomical features or non-anatomical objects and notification configurations. For example, the system 110 may be configured to obtain an input from a user of the surgical imaging system, where the input comprises a mapping between at least one anatomical feature or non-anatomical object and at least one notification configuration. The system may be configured to select a notification configuration, e.g., either the first notification configuration or the second notification configuration, based on the mapping.

In many cases, it is not useful to display a notification for every distance between every anatomical feature or object and every surgical tool. Instead, notifications may be provided if the distance crosses a threshold. In other words, the system may be configured to provide the notification based on a comparison of the distance with a distance threshold, such as a distance threshold of at most 40 mm (or at most 30 mm, or at most 20 mm, or at most 10 mm). For example, the notification may be provided if the distance falls below the distance threshold. Different distance thresholds may be used for different categories of anatomical features, non-anatomical objects and/or surgical tools. Object detection may be used, where Al (i.e., a machine learning model) detects and identifies different surgical instruments, adjusting feedback accordingly. In other words, the system may be configured to use different distance thresholds for different categories of surgical tools, anatomical features or non-anatomical objects.

The proposed concept may provide real-time distance measurements between surgical tools and critical sub-surface structures using an Image-Guided Surgery (IGS) system. (Multiple) feedback modalities, such as audio, visual, and haptic alerts, may be used to inform surgeons about the proximity of tools. The proposed concept may integrate IGS, Al-based tool detection, and proximity alerts. In the proposed concept, the system may change instrument visualization, such as color changes, when approaching critical structures. For example, the system may provide feedback even when the tool tip is obscured or immersed in tissue.

In the proposed concept, the system 110 may be used for the purpose of generating a digital view of the surgical site. This digital view is generated using image data of the surgical imaging device, e.g., by performing image processing on the image data of the optical imaging sensor(s) of the surgical imaging device. In addition, the aforementioned overlay can be overlaid over the view of the surgical site. The digital view is then provided to a display device, such as ocular displays 130a, a stereo head-mounted display or a monitor 130b. This digital view (e.g., of the surgical site) may be created and provided to the display device of the surgical imaging system as part of a display signal. In other words, the system may be configured to output a display signal to the display device 130a, 130b of the surgical or scientific imaging system, with the display signal comprising the (digital) view on the surgical site. The view may be viewed by the user, e.g., the surgeon, of the surgical imaging system. For example, the display signal may be a signal for driving (e.g., controlling) the respective display device. For example, the display signal may comprise video data and/or control instructions for driving the display. For example, the display signal may be provided via one of the one or more interfaces 112 of the system. Accordingly, the system 110 may comprise a video interface 112 that is suitable for providing the display signal to the display device 130a, 130b of the microscope system 100.

In the proposed surgical imaging system, optical imaging sensor(s) of the surgical imaging device 130 (or additional optical imaging sensors 150) are used to provide the aforementioned image data. Accordingly, the optical imaging sensor(s), which may be part of the imaging device 120 (e.g., of the microscope) may be configured to generate the image data. For example, the optical imaging sensor(s) of the imaging device 120 may comprise or be an APS (Active Pixel Sensor)-based imaging sensor or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensor by circuitry of the imaging sensor to perform the imaging.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system 110 and of the surgical imaging system 100 are mentioned in connection with the proposed concept or one or more examples described above or below (e.g., Figs. 2 to 4). The system 110 and the surgical imaging system 100 may comprise one or more additional optional features corresponding to one or more aspects of the proposed concept or one or more examples described above or below.

Some aspects of the present disclosure relate to a method for a surgical imaging system 100. For example, the method may be performed by the surgical imaging system 100 shown in Fig. 1b, and in particular by the system 110 shown in Figs. 1a and 1b. The method comprises obtaining 310 image-guided surgery data of an image-guided surgery system 50. The image-guided surgery data comprises three-dimensional anatomical information of a patient. The method comprises obtaining 320 image data of at least one optical imaging sensor of the surgical imaging system. The image data shows a surgical site 10 of the patient and at least one surgical tool 20. The method comprises determining 330 a position of a portion of the surgical tool relative to the surgical site based on the image data. The method comprises determining 340 a distance between the position of the portion of the surgical tool and at least one anatomical feature 30 of the patient based on the image-guided surgery data. The method comprises providing 350 a notification based on the distance between the position of the portion of the surgical tool and at least one anatomical feature of the patient.

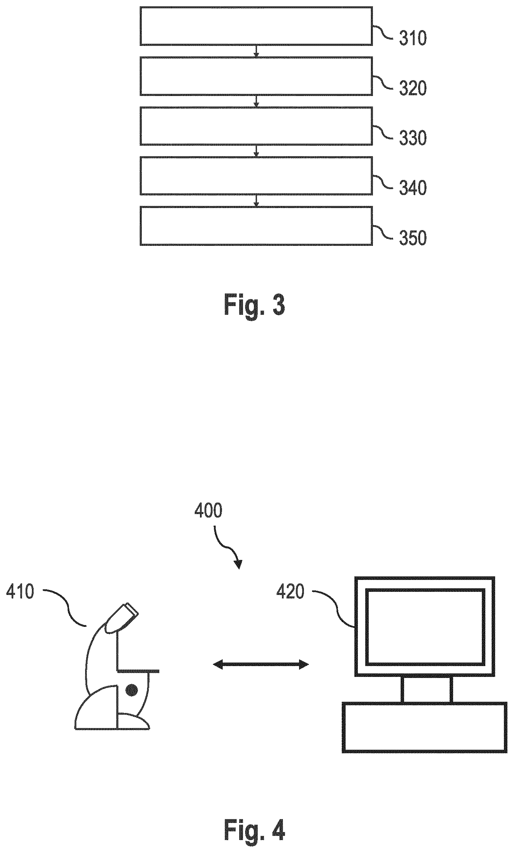

Features discussed in connection with the surgical imaging system of Figs. 1a to 2 may likewise be included in the method of Fig. 3.

More details and aspects of the method are mentioned in connection with the proposed concept, or one or more examples described above or below (e.g., Fig. 1a to 2, 4). The method may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device comprising a system as described in connection with one or more of the Figs. 1a to 3. Alternatively, an imaging device, such as a microscope, exoscope or endoscope, may be part of or connected to a system as described in connection with one or more of the Figs. 1a to 3. Fig. 4 shows a schematic illustration of a system 400 configured to perform a method described herein. The system 400 comprises an imaging device 410 and a computer system 420. The imaging device 410 is configured to take images and is connected to the computer system 420. The computer system 420 is configured to execute at least a part of a method described herein. The computer system 420 may be configured to execute a machine learning algorithm. The computer system 420 and imaging device 410 may be separate entities but can also be integrated together in one common housing. The computer system 420 may be part of a central processing system of the imaging device 410 and/or the computer system 420 may be part of a subcomponent of the imaging device 410, such as a sensor, an actor, a camera or an illumination unit, etc. of the imaging device 410.

The computer system 420 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 420 may comprise any circuit or combination of circuits. In one embodiment, the computer system 420 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a imaging device or a imaging device component (e.g. camera) or any other type of processor or processing circuit. Other types of circuits that may be included in the computer system 420 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 420 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 420 may also include a display device, one or more speakers, and a keyboard and/or controller, which can include a mouse, trackball, touch screen, voice-recognition device, or any other device that permits a system user to input information into and receive information from the computer system 420.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all the functionalities of the methods described herein. In some embodiments, a field programmable gate array may cooperate with a microprocessor to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 10: Surgical site
- 20: Surgical tool
- 30: Anatomical feature
- 40a, 40b: Visual notification
- 50: IGS system
- 100: Surgical imaging system
- 110: System
- 120: Surgical imaging device
- 130a: Ocular displays
- 130b: Monitor
- 210: Critical structures, tumor
- 220: Tip of suction device
- 230: Arrow
- 240: Tip of forceps
- 250: Notification icon
- 260: Highlighted border
- 310: Obtaining IGS data
- 320: Obtaining image data
- 330: Determining a position of a portion of a surgical tool
- 340: Determining a distance
- 350: Providing a notification
- 400: System
- 410: Imaging device
- 420: Computer system

## Claims

1. A system (110) for a surgical imaging system (100), the system comprising one or more processors and one or more storage devices, wherein the system is configured to:
obtain image-guided surgery data of an image-guided surgery system (50), the image-guided surgery data comprising three-dimensional anatomical information of a patient; obtain image data of at least one optical imaging sensor of the surgical imaging system, the image data showing a surgical site (10) of the patient and at least one surgical tool (20);
determine a position of a portion of the surgical tool relative to the surgical site based on the image data;
determine a distance between the position of the portion of the surgical tool and at least one anatomical feature (30) of the patient based on the image-guided surgery data; and
provide a notification (40a, 40b) based on the distance between the position of the portion of the surgical tool and at least one anatomical feature of the patient.

2. The system according to claim 1, wherein the system is configured to determine the distance between the position of the portion of the surgical tool and at least one anatomical feature of the patient being located below a surface of the surgical site based on the image-guided surgery data.

3. The system according to claim 2, wherein the system is configured to determine the position of the portion of the surgical tool regardless of the portion of the surgical tool being above or below the surface of the surgical site.

4. The system according to one of the claims 1 to 3, wherein the system is configured to determine a position of a non-anatomical object relative to the surgical site based on the image data, determine a distance between the position of the portion of the surgical tool and the position of the non-anatomical object, and provide the notification based on the distance between the position of the portion of the surgical tool and the position of the non-anatomical object.

5. The system according to one of the claims 1 to 4, wherein the system is configured to provide a notification having a first notification configuration based on the distance between the position of the portion of the surgical tool and a first anatomical feature and a second notification having a second notification configuration based on the distance between the position of the portion of the surgical tool and a second anatomical feature.

6. The system according to claim 5, wherein the system is configured to select one of the first notification configuration or the second notification configuration based on a category of the respective surgical tool, anatomical feature or non-anatomical object, the category being one of a plurality of pre-defined categories.

7. The system according to claim 6, wherein the plurality of pre-defined categories comprises at least a first category being used for tissue to be removed and a second category being used for blood vessels.

8. The system according to claim 5, wherein the system is configured to obtain an input from a user of the surgical imaging system, the input comprising a mapping between at least one anatomical feature and at least one notification configuration, and to select one of the first notification configuration or the second notification configuration based on the mapping.

9. The system according to one of the claims 1 to 8, wherein the system is configured to vary at least one of an intensity, a size or a transparency of the notification based on the distance,
and/or wherein the system is configured to provide the notification based on a comparison of the distance with a distance threshold.

10. The system according to one of the claims 1 to 9, wherein the system is configured to generate a view on the surgical site based on the image data, the view including an overlay with the notification, and provide the view on the surgical site for a display device of the surgical imaging system.

11. The system according to claim 10, wherein the system is configured to include the overlay in the view on the surgical site based on a comparison of the distance with a distance threshold,
and/or wherein a shape of the overlay is based on at least one of a shape or projected shape of the surgical tool or a projected shape of the anatomical feature.

12. The system according to one of the claims 1 to 11, wherein the system is configured to provide an audio output comprising the notification,
and/or wherein the system is configured to provide a control signal for a haptic notification device, with the control signal comprising the notification.

13. The system according to one of the claims 1 to 12, wherein the system is configured to process the image data using a machine-learning model to estimate a pose of the surgical tool, and to determine the position of the portion of the surgical tool based on the estimated pose of the surgical tool,
and/or wherein the system is configured to determine the position of the portion of the surgical tool by fitting a projection of a three-dimensional model of the surgical tool to the surgical tool shown in the image data.

14. A method for a surgical imaging system (100), the method comprising:
obtaining (310) image-guided surgery data of an image-guided surgery system (50), the image-guided surgery data comprising three-dimensional anatomical information of a patient;
obtaining (320) image data of at least one optical imaging sensor of the surgical imaging system, the image data showing a surgical site (10) of the patient and at least one surgical tool (20);
determining (330) a position of a portion of the surgical tool relative to the surgical site based on the image data;
determining (340) a distance between the position of the portion of the surgical tool and at least one anatomical feature (30) of the patient based on the image-guided surgery data; and
providing (350) a notification based on the distance between the position of the portion of the surgical tool and at least one anatomical feature of the patient.

15. A computer program having a program code for performing a method according to claim 14 when the program is executed on processor.
